# EUROPEAN PATENT APPLICATION

(11) **EP 2 851 010 A1**
(43) Date of publication of application: **25.03.2015**
(21) Application number: 13743591.3
(22) Date of filing: 23.01.2013
(51) Int. Cl.: A61B 17/11, A61B 17/064, A61B 17/08, A61B 17/10

(54) **VASCULAR ANASTOMOSIS DEVICE AND VASCULAR ANASTOMOSIS METHOD**

(30) Priority: 30.01.2012 JP 2012016272
(71) Applicant: Kurume University, Kurume-shi Fukuoka 830-0011 (JP); Nhk Spring Co., Ltd., Kanazawa-ku Yokohama-shi Kanagawa 236-0004 (JP)
(72) Inventor: WATANABE, Koichi, Kurume-shi Fukuoka 830-0011 (JP)
(74) Representative: Kalhammer, Georg
(86) International application number: PCT/JP2013/051258
(87) International publication number: WO 2013/115022

(57) **Abstract**

Provided is a vascular anastomosis device for favorably anastomosing two blood vessels with different diameters. T he vascular anastomosis device comprises two rings with pins 21 made of a flexible material and an openable ring holder 2 2, which is made of a pair of components and detachably hold s the rings with pins 21, the ring holder 22 has a means for deforming the rings with pins, the end portion of the large-diameter blood vessel is inserted through a ring 21 and the vascular wall is extended outward 90 degrees and impaled on the pins, the end portion of the small-diameter blood vessel is inserted through the other ring 21, an incision is made i n a longitudinal direction from the end surface of the small -diameter blood vessel, the two rings 21 are elliptically de formed into a same elliptical shape by the means for deformi ng the rings so that the minor axis agrees with the diameter of the small-diameter blood vessel, the small-diameter blood vessel is elliptically opened and the vascular wall is exte nded outward 90 degrees and impaled on the pins, and the two rings 21 are joined together by allowing the pins provided o n the ring in one component to stick into the ring in the oth er component.

## Description

### Technical Field

The present invention relates to a vascular anastomosis device, which is a device for connecting and conglutinating two blood vessels with different diameters.

### Background Art

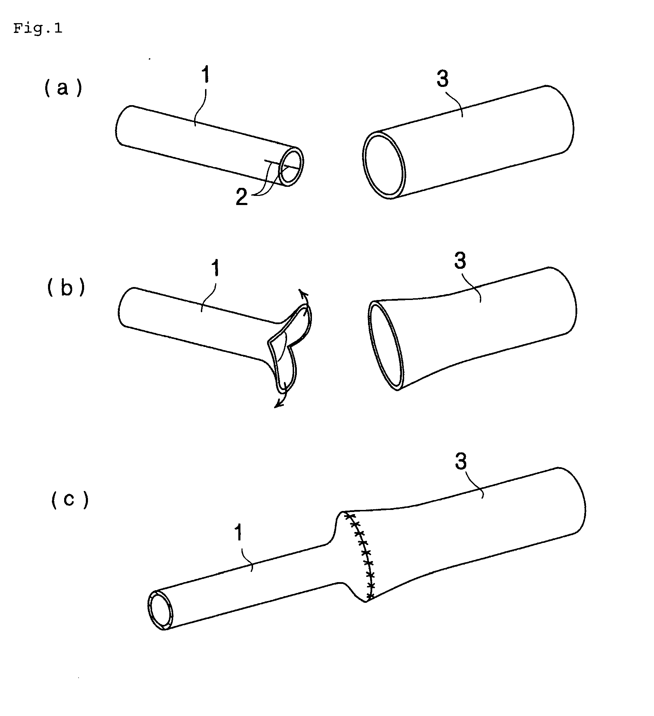

Conventionally, for connecting and conglutinating two blood vessels, suturing or anastomosis using an anastomosis device has been performed. In the cases where the two blood vessels have different diameters, however, the only option is suturing. For anastomosis of blood vessels with different diameters, it is necessary that the two end surfaces to be coupled be made identical in size. For the purpose, as shown in Fig. 1 (a), an incision 2 is made in the longitudinal direction from the end surface of the small-diameter blood vessel 1. Then, as shown in Fig. 1 (b), the small-diameter blood vessel 1 is opened so as to fit the size of the end surface of the large-diameter blood vessel 3. After that, as shown in Fig. 1 (c), the blood vessels 1 and 3 are sutured in the state where the end surfaces are joined together. However, connecting and conglutinating blood vessels by suturing has a disadvantage of taking immense amount of time and effort.

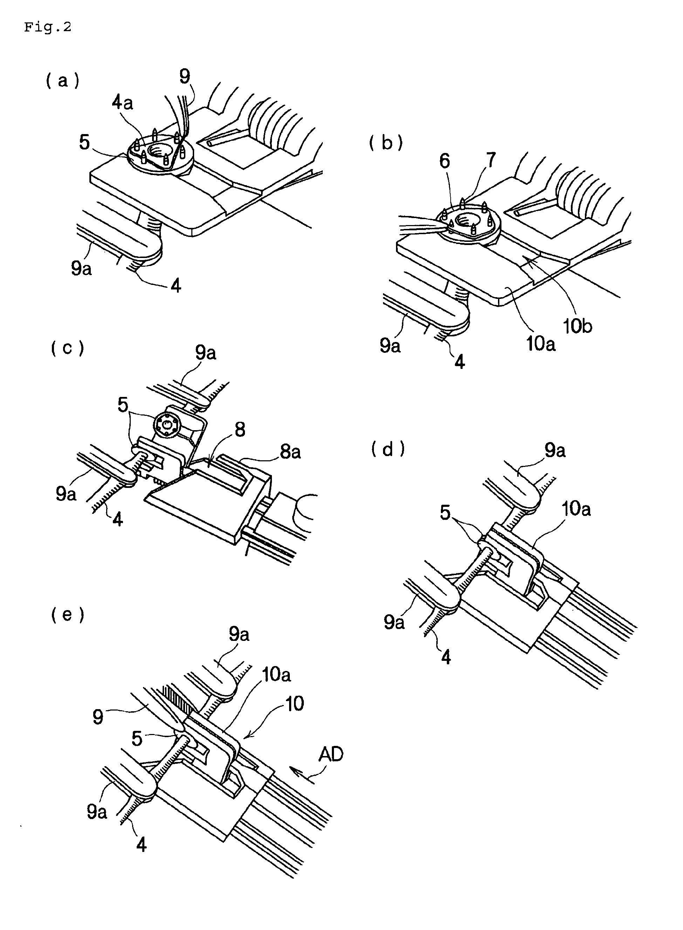

To overcome the disadvantage, an anastomosis device for anastomosing blood vessels by separately inserting blood vessels through truly circular rings with pins, pulling outward the end portion of each vessel and impaling it on the pins, and joining the two rings together was developed (refer to Non Patent Literature 1). Referring to Fig. 2, anastomosis using a conventional vascular anastomosis device will be described. After a blood vessel end portion 4a held with forceps 9 is inserted through a truly circular ring 5 as shown in Fig. 2 (a), a vascular wall 6 is extended outward 90 degrees and impaled on pins 7 as shown in Fig. 2 (b). The other blood vessel is also treated in the same manner. Next, as shown in Fig. 2 (c), while the two rings 5 are moved closer to each other, the knob (not shown) of the anastomosis device is turned to introduce the two rings 5 into the guide slit 8. Thus the rings 5 are joined together as shown in Fig. 2 (d). Further, as shown in Fig. 2 (e), the rings 5 are tightly clamped with forceps 9 so that the rings are completely joined. After that, further turning the knob allows a protrusion 8a (refer to Fig. 2 (c)) to advance into the guide groove 10b provided on the guide plate 10a shown in Fig. 2 (b) (refer to the arrow direction AD in Fig. 2 (e)) and push the rings 5 so that the rings 5 are released from the anastomosis device 10. A pair of tweezers 9a is used for holding the blood vessel 4.

In the cases where blood vessels with almost the same diameters are subjected to anastomosis, the anastomosis device shown in Fig. 2 can be used satisfactorily. However, since the conventional anastomosis device as shown in Fig. 2 uses two truly circular rings, the device has disadvantages shown below in connecting blood vessels with different diameters. That is, if rings suitable for the large diameter blood vessel are used, the small-diameter blood vessel needs to be forcibly adapted to the large ring, which can result in unsuccessful anastomosis due to wall tearing of the small-diameter blood vessel. Conversely, if rings suitable for the small-diameter blood vessel are used, anastomosis will be impossible due to too loose fitting for the large-diameter blood vessel.

### Citation List

### Non Patent Literature

### Non Patent Literature 1:

Kiso to Rinsho (The Clinical Report) "Bishoukekkann hunngouki 'Precise' shiyousha no shugiteki tekiou ni kannsuru ichi kousatsu (Technical Indications for Surgeons of Microvascular Anastomotic Device 'Precise')", 27(13): 5393-5397, 1993, 319-323

### Summary of Invention

### Technical Problem

The present invention was made in view of such a problem of the prior art, and an objective thereof is to provide a vascular anastomosis device and a vascular anastomosis method which enable favorable anastomosis of blood vessels with different diameters.

### Solution to Problem

To solve the above problem, the present invention utilizes the suturing shown in Fig. 1 (b). That is, the present inventor focused attention on the fact that, in the suturing where the small-diameter blood vessel 1 is opened so as to fit the size of the end surface of the large-diameter blood vessel 3, the end surface of the small-diameter blood vessel becomes elliptical in shape. In the present invention, in the cases where blood vessels are anastomosed using the rings as shown in Fig. 2, a flexible material is employed as the material for the ring through which the small-diameter blood vessel is inserted and the ring through which the large-diameter blood vessel is inserted. Hereinafter, the concept of the vascular anastomosis method using the vascular anastomosis device of the present invention will be described referring to Figs. 3 (a) to (f).

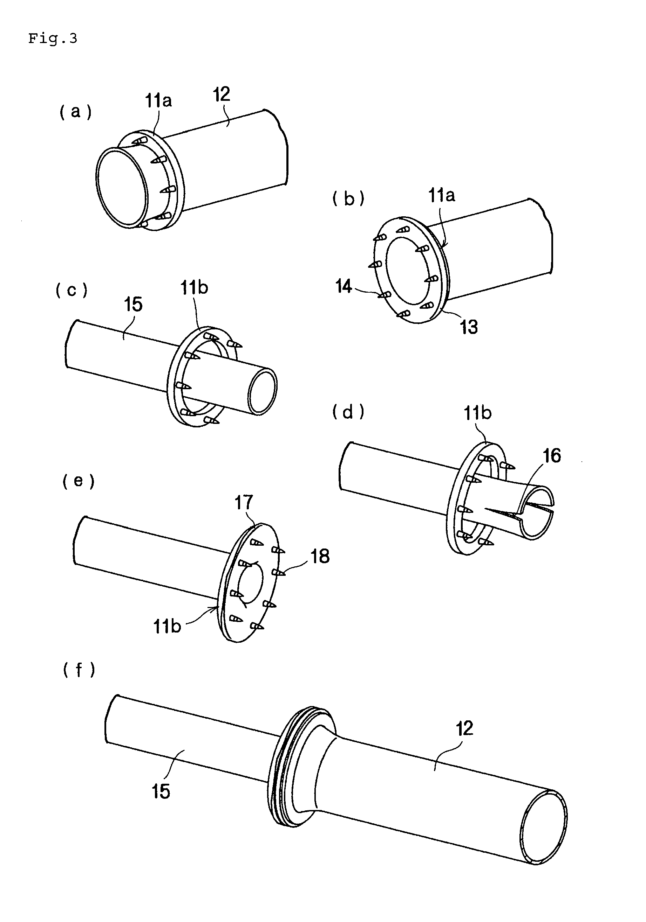

First, as shown in Fig. 3 (a), a ring 11a, which has pins and is suitable for the large-diameter blood vessel, is selected and the end portion of the large-diameter blood vessel 12 is inserted through the ring. Then, as shown in Fig. 3 (b), the vascular wall 13 is extended outward 90 degrees and impaled on the pins 14. Next, as shown in Fig. 3 (c), the end portion of the small-diameter blood vessel 15 is inserted through the ring 11b, which has pins and is of the same size as the above ring 11a. As shown in Fig. 3 (d), the ring 11b is elliptically deformed so that the minor axis agrees with the diameter of the small-diameter blood vessel, and an incision 16 is made in the longitudinal direction from the end surface of the small-diameter blood vessel 15. Further, the ring 11a shown in Fig. 3 (b) is deformed into the elliptical shape of the ring 11b shown in Fig. 3 (d). Then, as shown in Fig. 3 (e), the small-diameter blood vessel 15 is elliptically opened, and the vascular wall 17 is extended outward 90 degrees and impaled on pins 18. Subsequently, the two rings 11a and 11b are joined together as shown in Fig. 3 (f) so that the large diameter blood vessel 12 and the small-diameter blood vessel 15 are anastomosed.

That is, the vascular anastomosis device of the present invention is a vascular anastomosis device for anastomosing two blood vessels with different diameters, characterized in that the device comprises a first ring with pins and a second ring with pins both made of a material which is deformable by an external force and allows the pins to stick thereinto, and an openable ring holder, which is made of a pair of components, detachably holds the first ring and the second ring, and has a means for deforming the rings with pins, and that vascular anastomosis can be achieved in the following manner: the ring holder holding the rings is opened, the end portion of the large-diameter blood vessel is inserted through the first ring and the vascular wall is extended outward 90 degrees and impaled on the pins, the end portion of the small-diameter blood vessel is inserted through the second ring, an incision is made in a longitudinal direction from the end surface of the small-diameter blood vessel, the first ring and the second ring are deformed into a same elliptical shape by the means for deforming the rings so that the minor axis agrees with the diameter of the small-diameter blood vessel, the small-diameter blood vessel is elliptically opened and the vascular wall is extended outward 90 degrees and impaled on the pins, and the first ring and the second ring are joined together to allow the pins provided on the ring in one component to stick into the ring in the other component and anastomose the large-diameter blood vessel and the small diameter blood vessel.

In addition, the vascular anastomosis method of the present invention is a vascular anastomosis method for anastomosing two blood vessels with different diameters, characterized in that a first ring with pins and a second ring with pins both made of a material which is deformable by an external force and allows the pins to stick thereinto are provided, and that the end portion of the large-diameter blood vessel is inserted through the first ring and the vascular wall is extended outward 90 degrees and impaled on the pins, the end portion of the small-diameter blood vessel is inserted through the second ring, an incision is made in a longitudinal direction from the end surface of the small-diameter blood vessel, the first ring and the second ring are deformed into a same elliptical shape by a means for deforming the rings so that the minor axis agrees with the diameter of the small-diameter blood vessel, the small-diameter blood vessel is elliptically opened and the vascular wall is extended outward 90 degrees and impaled on the pins, and the first ring and the second ring are joined together to allow the pins provided on the ring in one component to stick into the ring in the other component and anastomose the large-diameter blood vessel and the small diameter blood vessel.

### Advantageous Effects of Invention

By using the vascular anastomosis device and the vascular anastomosis method of the present invention, blood vessels with different diameters can be anastomosed. Since the rings with pins are made of a material which is deformable by an external force and allows the pins to stick thereinto, vascular obstruction due to collapse can be prevented and flexible expansion can be achieved when blood flow is increased.

### Brief Description of Drawings

Figs. 1(a) to (c) are figures for illustrating a vascular anastomosis method by hand suturing.
Figs. 2(a) to (e) are figures for illustrating a vascular anastomosis method using a conventional vascular anastomosis device.
Figs. 3(a) to (f) are figures for illustrating the concept of the vascular anastomosis method using the vascular anastomosis device of the present invention.

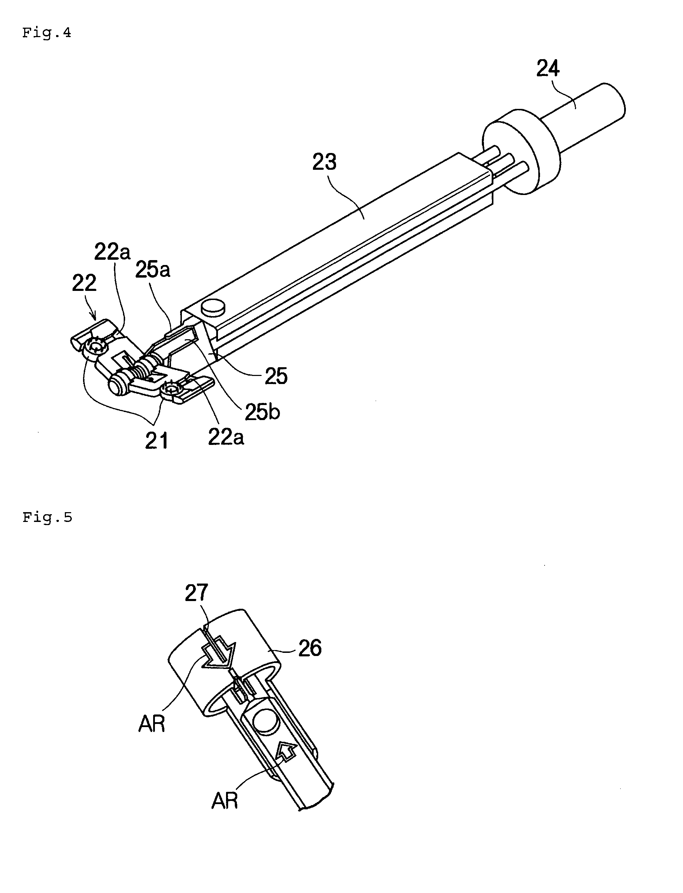

Fig. 4 is a perspective view showing an embodiment of the vascular anastomosis device of the present invention.
Fig. 5 is a perspective view for illustrating uncapping of the cartridge at the tip of the vascular anastomosis device of the present invention.

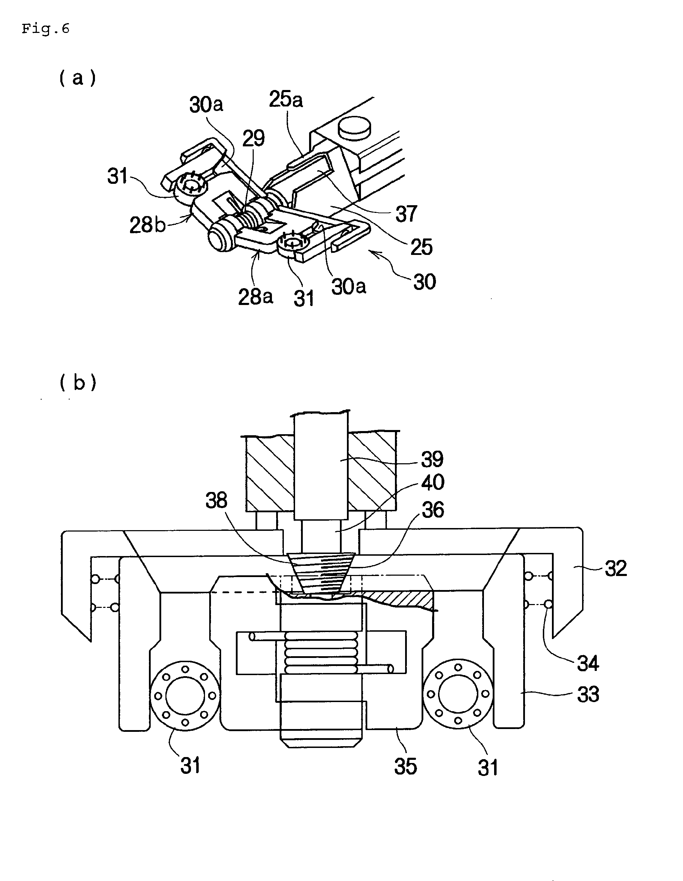

Fig. 6 (a) is a perspective view showing Example 1 of the vascular anastomosis device of the present invention, and Fig. 6 (b) is a partial plan view showing the connection diagram of the shaft portion as the rotation center of the cartridge of Fig 6 (a).

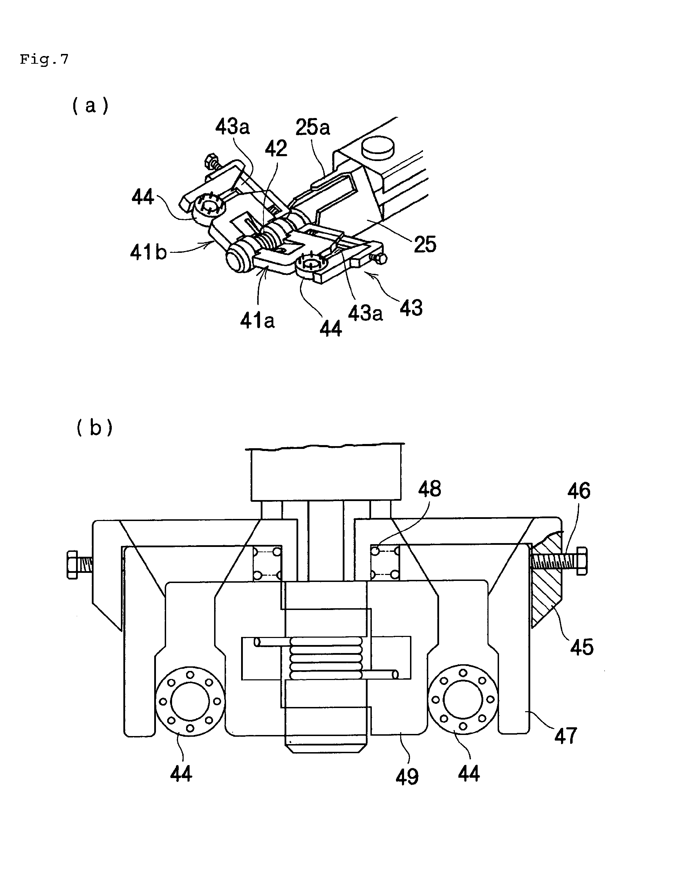

Fig. 7 (a) is a perspective view showing Example 2 of the vascular anastomosis device of the present invention, and Fig. 7 (b) is a partial plan view showing the connection diagram of the shaft portion as the center of the cartridge of Fig 7 (a).

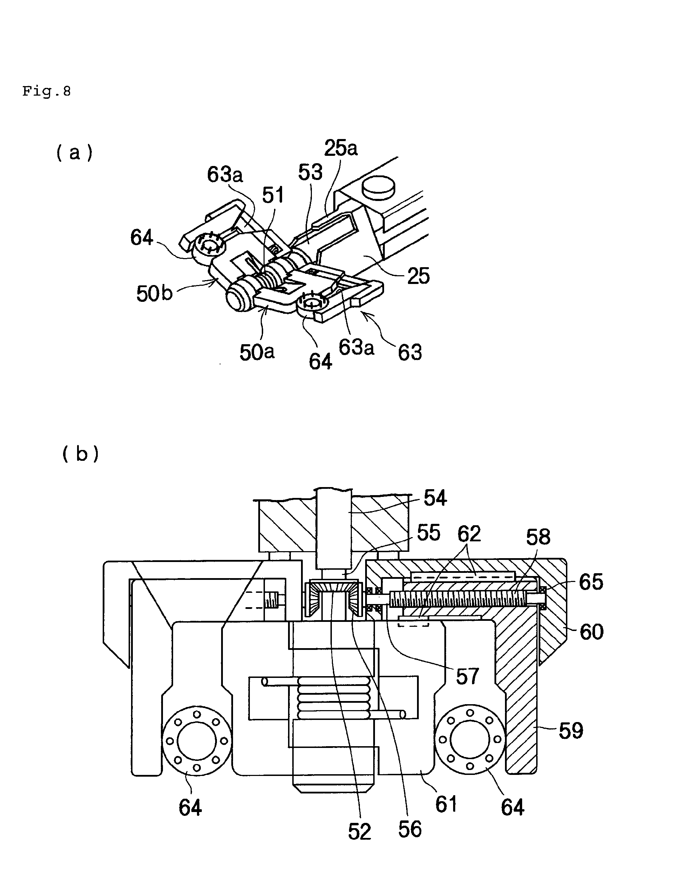

Fig. 8 (a) is a perspective view showing Example 3 of the vascular anastomosis device of the present invention, and Fig. 8 (b) is a partial plan view showing the connection diagram of the shaft portion as the rotation center of the cartridge of Fig 8 (a).

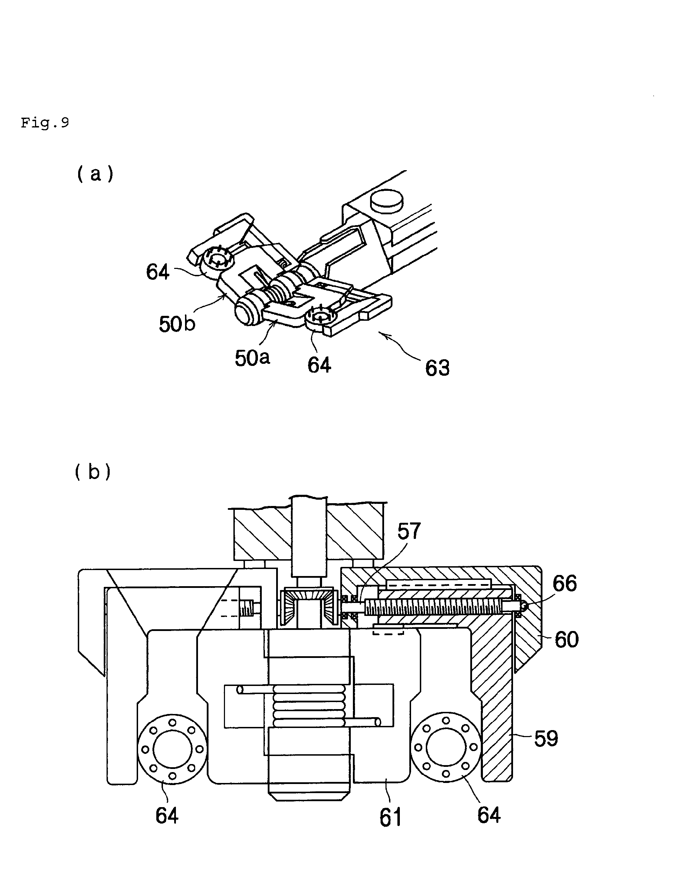

Fig. 9 is a view showing a modification example of Fig. 8.

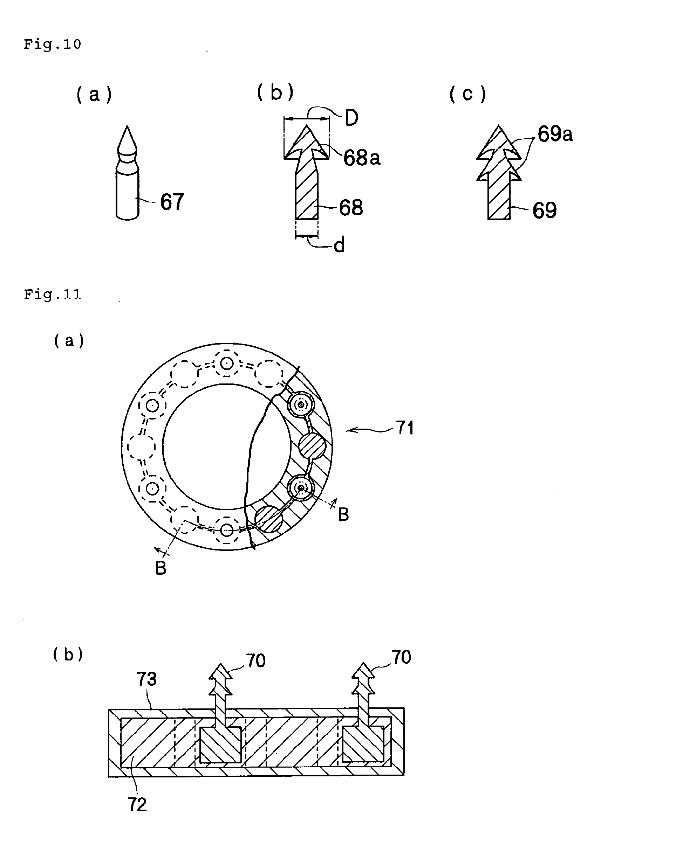

Fig. 10 (a) is a front view showing the pin shape in a conventional ring with pins, and Figs. 10 (b) and (c) are front views showing separate examples of the ring with pins of the present invention.
Fig. 11 (a) is a plan view of an example of the ring with pins of the present invention, and Fig. 11 (b) is a sectional view along arrows B-B in Fig. 11 (a).

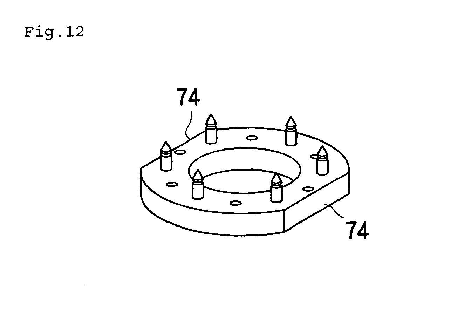

Fig. 12 is a perspective view showing another example of the ring with pins of the present invention.

### Description of Embodiments

Hereinafter, embodiments of the present invention will be described. Needless to say, other embodiments can also be used without departing from the scope of the invention.

### (1) Shape of Ring

The ring before deformation may be truly circular or elliptical, and needs to be flexible. When two blood vessels with different diameters are anastomosed, the diameter of the rings needs to fit the larger blood vessel. If the diameter of the rings is smaller than that of the larger blood vessel, anastomosis will be impossible due to too loose fitting for the large-diameter blood vessel. Conversely, if the diameter of the rings is too large, the small-diameter blood vessel needs to be forcibly adapted to the large ring, which can result in wall tearing of the small-diameter blood vessel. As mentioned above, the rings are deformed into an elliptical shape. As will be described later in the section of Deformation Mechanism, there is a possibility that efficient joining of the two rings cannot be achieved due to ring slippage that can occur while a predetermined force for elliptical deformation is transmitted to the ring. For efficient force transmission to both sides of each ring, and for the prevention of slippage or falling of the ring during the deformation, it is recommended that the flat surface 74 perpendicular to the direction of the force is provided on the outer side surface of the ring, as shown in Fig. 12. In the present invention, the term ellipse includes not only mathematically defined ellipses (the set of points such that the sum of the distances to two fixed points (the foci) is constant) but also wide variety of ovals (shapes like a circle, but wider in one direction than the other).

Next, the dimension of the rings will be described. The dimension and the shape of the rings used in the present invention are not particularly limited and a ring of any size and shape may be used as long as vascular anastomosis can be efficiently achieved using the rings. However, since blood vessels with different diameters are anastomosed in the present invention, the rings need to have a flange-like shape and a diameter which fits the large-diameter blood vessel, as described above. In this view, commercially available standard rings can be used, but other rings than those in the standard range are also usable in principle. It is recommended that, in the cases where elliptical rings are used, the rings have such a diameter that the long-diameter blood vessel can effortlessly be inserted therethrough. In particular, regarding commercially available ordinary rings for anastomosing blood vessels of the same diameter, those having inner diameters of 1.0 to 3.0 mm are widely used, and in the present invention as well, the inner diameter (in the case of an ellipse, the inner shorter diameter; hereinafter referred to as the "inner minor axis") may be suitably used as described above. However, the vascular anastomosis device of the present invention can also be applied to larger blood vessels, for example the human aorta, of which the diameter is 40 mm or more. Too fine blood vessels (for example, capillaries) cannot be anastomosed or do not need anastomosis in principle. Therefore, the range of the ring inner diameter (in the case of an elliptic ring, the inner minor axis) of 0.3 to 50 mm is sufficient for most human blood vessels. When applied to the anastomosis of blood vessels of large animals, further larger inner diameters may be used. The width of the ring flange (half of the difference between the outer diameter and the inner diameter) is also not particularly limited, and any ring having a similar flange width to that of a commercially available conventional ring can be used without any problem. However, it is recommended that the flange width is determined as appropriate depending on the diameter of the blood vessel to be inserted and the shape of the pins. Specifically, the generally recommended flange width of the ring is about 0.3 to 10 mm.

Further, the thickness of the rings used in the present invention is not particularly limited and has only to allow the pins provided on one ring to stick into the other ring when the rings are joined and to prevent the pins from being easilypulled out. Specifically, when applied to human blood vessels as described above, the recommended thickness is 0.3 to 20 mm, in particular 0.5 to 10 mm. When applied to blood vessels with larger diameters, for example to blood vessels of large animals, the thickness may be more.

As will be described later, the ring body of the present invention made of a flexible material may be thicker and the pins may be longer, as compared to conventional commercial rings and pins, to some degree in order that pins are more securely caught in the opposing ring in the joined state of the rings. Too long pins will stick through the opposing ring and protrude from the other side, causing problems. Too small dimensions (including the thickness) of the rings can prevent efficient vascular anastomosis and too large dimensions can cause adverse effects on the patient after operation.

### (2) Flexible Materials

The rings generally used for conventional anastomosis devices are made of polyethylene resins, which are not flexible. Materials that can be used for the rings of the present invention are deformable by an external force and allow the pins to stick thereinto, and as such materials, flexible materials can be used. The materials preferably have biocompatibility as well. Specific examples of the materials that can be used for the rings of the present invention include a silicone resin, a natural rubber, a synthetic rubber, a flexible epoxy resin, a thermoplastic elastomer, an aliphatic polyurethane, a polyether block amide copolymer, and a flexible fluororesin. However, materials usable in the present invention are not limited to these. It is preferable that the ring made of such a flexible material easily deforms when a predetermined force is applied, and it is more preferable that the ring can be restored to the original shape when the force for deformation is removed. The reason is that, in the course of anastomosis of blood vessels with different diameters, even if the force for deformation is so strong that the ring deforms into an unfavorable shape, removal of the force restores the ring to the original shape, allowing another deformation.

### (3) Ring Deformation Mechanism

### Overall Configuration

Fig. 4 is a perspective view showing an embodiment of the vascular anastomosis device of the present invention. The vascular anastomosis device comprises two identical rings with pins 21 made of a flexible material, an openable ring holder 22, which detachably holds the rings with pins 21, and a sliding mechanism comprising a cylinder 23 for supporting the ring holder 22 so as to allow the ring holder to move back and forth, and a rotary knob 24. The ring holder 22 may have a wide variety of means for deforming the rings as will be described later. While the two rings 21 are brought closer to each other by closing movement of the ring holder 22, the rotary knob 24 is turned to introduce the two rings 21 into the guide slit 25b and join the two rings 21. After that, further turning the rotary knob 24 allows the support bar 25 to extend so that the protrusion 25a advances into the guide groove 22a provided on the ring holder 22 and pushes the rings 21 so that the rings 21 are released from the ring holder 22.

### Example 1

A locking hook (not shown) provided on the cylinder 23 is engaged in the slot 27 of the cap 26, and thereby the cartridge at the tip of the vascular anastomosis device is protected with the cap 26, as shown in Fig. 5. The cap 26 is pinched between fingers and removed. Fig. 4 shows the state where the cap 26 has been removed in this way. The arrows AR in Fig. 5 show the insertion direction of the cartridge at the tip of the vascular anastomosis device into the cap 26.

As shown in Fig. 6 (a), the cartridge comprises a pair of right and left components 28a and 28b. The components 28a and 28b are held around the shaft with use of a spring 29, and therefore, when the cap 26 is removed, the spring force opens the cartridge. The ring 31 held by the ring holder 30 in the cartridge component 28a is, as shown in Fig. 6 (b), subjected to a force in the direction toward the fixed retainer plate 35 due to the resilience of the spring 34 held between the outer frame 32 and the movable retainer plate 33. Inside the movable retainer plate 33, a tapered threaded portion 36 is provided. There is no intention to provide detailed description, but the other cartridge component 28b has the same configuration, that is, comprises the ring holder 30, the ring with pins 31, the outer frame 32, the movable retainer plate 33, the spring 34, the fixed retainer plate 35, and the tapered threaded portion 36.

The diameter of the rings 31 fits the large-diameter blood vessel. The large-diameter blood vessel is inserted through either of the right and left rings 31 and the vascular wall is impaled on the pins of the ring, as shown in Fig. 3(b).

The shaft as the rotation center of the cartridge is, as shown in Fig. 6 (b), provided with a tapered threaded portion 38 which screws with the tapered threaded portion 36. The tip of the rotatable driver 39, which passes through the bar 37 (refer to Fig. 6 (a)) held by the cylinder 23 (refer to Fig. 4), is capable of engaging with the protrusion 40 provided at the end of the threaded portion 38.

Next, the small-diameter blood vessel is inserted through the other ring 31, and the tip of the driver 39 is rotated in an engaged state with the protrusion 40 to move the threaded portion 38 in the upward direction in the figure. As a result, the movable retainer plate 33 is subjected to a force in the direction toward the fixed retainer plate 35 due to the resilience of the spring 34, and the ring 31 is deformed into an elliptical shape. The driver 39 is continuously moved until the minor axis of the deformed rings 31 corresponds to the diameter of the small-diameter blood vessel. Since the deformation of the rings 31 by the operation of the driver 39 occurs in the same manner in both the right and left cartridge components, the two rings 31 are deformed into the same elliptical shape. Then, an incision is made in a longitudinal direction from the end surface of the small-diameter blood vessel, the small-diameter blood vessel is elliptically opened, and the vascular wall is extended outward 90 degrees and impaled on the pins (refer to Figs. 3(c) to 3 (e)). In this case, the operation of the driver deforms the two rings simultaneously and to the same degree.

The right and left cartridge components 28a and 28b are joined to allow the pins provided on the ring in one component to stick into the ring in the other component. Thus, the vascular anastomosis is completed (refer to Fig. 3 (f)). After that, the driver 39 is moved in the downward direction in the figure to reduce the resilience of the spring 34 until the driver is returned to its original position. Further, as described in paragraph [0018], turning the rotary knob 24 shown in Fig. 4 allows the support bar 25 to extend so that the protrusion 25a advances into the guide groove 30a provided on the ring holder 30 shown in Fig. 6 (a) and pushes the rings 31 so that the rings 31 are released from the ring holder 30. In order that the amount of movement of the driver 39 could be checked from the outside, an appropriate scale mark is preferably provided on the outside of the cylinder 23 (refer to Fig. 4). It is preferable that the screws or the like, which will be described later, are also provided with something equivalent to the scale mark.

### Example 2

As described above, the cartridge at the tip of the vascular anastomosis device is protected with a cap, which is to be removed in the same manner as above.

As shown in Fig. 7 (a), the cartridge comprises a pair of right and left components 41a and 41b. The components 41a and 41b are held around the shaft with use of a spring 42, and therefore, when the cap is removed, the spring force opens the cartridge. The ring 44 held by the ring holder 43 in the cartridge component 41a is, as shown in Fig. 7 (b), subjected to a force in the direction toward the fixed retainer plate 49 against the resilience of the spring 48 when the thread 46 passing through the outer frame 45 presses the movable retainer plate 47. There is no intention to provide detailed description, but the other cartridge component 41b has the same configuration, that is, comprises the ring holder 43, the ring with pins 44, the outer frame 45, the thread 46, the movable retainer plate 47, the spring 48, and the fixed retainer plate 49.

The diameter of the rings 44 fits the large-diameter blood vessel. The large-diameter blood vessel is inserted through either of the right and left rings 44 and the vascular wall is impaled on the pins of the ring, as shown in Fig. 3(b).

Next, the small-diameter blood vessel is inserted through the other ring 44, and the movable retainer plate 47 is pressed by the thread 46. As a result, a force in the direction from the movable retainer plate 47 toward the fixed retainer plate 49 against the resilience of the spring 48 generates, and allows the ring 44 to be deformed into an elliptical shape. The thread 46 is screwed until the minor axis of the deformed rings 44 corresponds to the diameter of the small-diameter blood vessel. Then, an incision is made in a longitudinal direction from the end surface of the small-diameter blood vessel, the small-diameter blood vessel is elliptically opened, and the vascular wall is extended outward 90 degrees and impaled on the pins (refer to Figs. 3(c) to 3 (e)).

The ring 44 through which the large-diameter blood vessel is inserted is pressed by screwing the thread 46 to the same degree as above, and thereby the two rings are deformed into the same elliptical shape. In this example, in order to equalize the deformation amounts of the two rings, it is recommended that scale marks be provided on the outer frame 45 so that the amount of pushed-in of the thread (or the amount of movement of the movable retainer plate 47) could be checked.

The right and left cartridge components 41a and 41b are joined to allow the pins provided on the ring in one component to stick into the ring in the other component. Thus, the vascular anastomosis is completed (refer to Fig. 3 (f)). After that, the thread 46 is pulled back outward so that the resilience of the spring 48 releases the movable retainer plate 47 from the pressed state and returns it to its original position. Further, as described in paragraph [0018], turning the rotary knob 24 shown in Fig. 4 allows the support bar 25 to extend so that the protrusion 25a advances into the guide groove 43a provided on the ring holder 43 shown in Fig. 7 (a) and pushes the rings 44 so that the rings 44 are released from the ring holder 43.

### Example 3

Here, the means for moving the movable retainer plate in Example 1 is replaced by bevel gears.

As described above, the cartridge at the tip of the vascular anastomosis device is housed in a cap, which is to be removed in the same manner as above.

As shown in Fig. 8 (a), the cartridge comprises a pair of right and left components 50a and 50b. The components 50a and 50b are held around the shaft with use of a spring 51, and therefore, when the cap is removed, the spring force opens the cartridge.

The shaft as the rotation center of the cartridge is, as shown in Fig. 8 (b), provided with a bevel gear 52. From the tip of the rotatable driver 54, which passes through the bar 53 (refer to Fig. 8 (a)) held by the cylinder 23 (refer to Fig. 4), an operation shaft 55 for the bevel gear 52 is protruded. In the cartridge component 50a, another bevel gear 56, which meshes with the bevel gear 52, is provided. A rod 57 is protruded from the bevel gear 56, and the rod 57 is provided with a threaded portion 58. The cartridge component 50a comprises a movable retainer plate 59, which comprises a threaded portion capable of meshing with the threaded portion 58 of the rod 57. The rod 57 is in contact with the outer frame 60, and the movement of the rod 57 to the right is prevented by the outer frame 60. The outer frame 60 and a fixed retainer plate designated by 61 have stoppers 62 for preventing the movable retainer plate 59 from rotating.

When the operation shaft 55 at the end of the rotatable driver 54 is operated to rotate the bevel gear 52, the bevel gear 56 meshing with the bevel gear 52 rotates and tries to send the rod 57 to the right, but the frame 60 prevents the movement. The counteraction forcibly sends the movable retainer plate 59 meshing with the threaded portion 58 of the rod 57 to the left. Since the threaded portion 58 of the rod 57 protruded from the bevel gear 56 meshes with the movable retainer plate 59, rotation of the bevel gear 56 could horizontally rotate the movable retainer plate 59. The stoppers 62, however, prevent the horizontal rotation. Thus, the counteraction resulting from the prevention of the movement of the rod 57 to the right, that is, the movement of the movable retainer plate 59 to the left exerts a force on the ring 64 held by the ring holder 63 in the cartridge component 50a in the direction toward the fixed retainer plate 61. The rod 57 is provided with the guide member 65. There is no intention to provide detailed description, but the other cartridge component 50b has the same configuration, that is, comprises the ring holder 63, the ring with pins 64, the bevel gear 56, the rod 57, the movable retainer plate 59, the outer frame 60, the fixed retainer plate 61, stoppers 62, and the guide member 65. In this example, the right and left movable retainer plates can move simultaneously and by the same amount.

The diameter of the rings 64 fits the large-diameter blood vessel. The large-diameter blood vessel is inserted through either of the right and left rings 64 and the vascular wall is impaled on the pins of the ring, as shown in Fig. 3(b).

After the small-diameter blood vessel is inserted through the other ring 64, the operation shaft 55 at the end of the rotatable driver 54 is operated to rotate the bevel gear 52. The bevel gear 56 meshing with the bevel gear 52 rotates and tries to send the rod 57 to the right, but the frame 60 prevents the movement. As the counteraction, the movement of the movable retainer plate 59 meshing with the threaded portion 58 of the rod 57 to the left exerts a force on the ring 64 held by the ring holder 63 in the cartridge component 50a in the direction toward the fixed retainer plate 61. Consequently, the ring 64 is deformed into an elliptical shape. The operation shaft 55 is operated until the minor axis of the deformed rings 64 corresponds to the diameter of the small-diameter blood vessel. Since the deformation of the rings 64 by the operation of the operation shaft 55 occurs in the same manner in both the right and left cartridge components, the two rings 64 are deformed into the same elliptical shape. Then, an incision is made in a longitudinal direction from the end surface of the small-diameter blood vessel, the small-diameter blood vessel is elliptically opened, and the vascular wall is extended outward 90 degrees and impaled on the pins (refer to Figs. 3 (c) to 3 (e)).

The right and left cartridge components 50a and 50b are joined to allow the pins provided on the ring in one component to stick into the ring in the other component. Thus, the vascular anastomosis is completed (refer to Fig. 3 (f)). After that, the operation shaft 55 is operated so that the bevel gear 52 rotates in the opposite direction to move the movable retainer plate 59 to the right. This movement releases the movable retainer plate 59 from the pressed state and returns it to its original position. Further, as described in paragraph [0018], turning the rotary knob 24 shown in Fig. 4 allows the support bar 25 to extend so that the protrusion 25a advances into the guide groove 63a provided on the ring holder 63 shown in Fig. 8 (a) and pushes the rings 64 so that the rings 64 are released from the ring holder 63.

### Example 4

In Example 4 , in order to reduce the friction at the contact site between the rod 57 and the outer frame 60 in Example 3, a ball bearing 66 is attached to the contact site between the rod 57 and the outer frame 60, as shown in Fig. 9 (b). Needless to say, other means can also be employed as long as the friction at the contact site can be reduced.

### Shape of Pins

In the cases where the rings with pins are made of a flexible material, the pin 67 having a conventional shape as shown in Fig. 10 (a) and being made of a certain type of material has the risk of being pulled out after stuck into the other ring. In such a case, it is preferred to adopt the pin 68 shown in Fig. 10 (b) or the pin 69 shown in Fig. 10 (c) each having a harpoon-like shape, that is, having a barb. The head portions 68a and 69a of the pins 68 and 69 are straight, but they may be curved. In the pins 68 and 69, the head diameter D must be larger than the shank diameter d, and the ratio of D to d is preferably about 1.5 or more.

### Other Ring Features

In a preferable example, the pins and the inner portion of the ring are made of a hard material (for example, polyethylene), and the outer portion and other portions of the ring are made of a flexible material (see, for example, paragraph [0017]). In a recommendable example, as shown in Fig. 11 (b), the head portions and the base portions of the pins 70 and the inner portion 72 (into which the pins of the other ring are to be inserted) of the ring 71 are made of a hard material, such as polyethylene, and the outer portion 73 and other portions of the ring 71 are made of a flexible material. Such a structure enables the head portions of the pin 70 made of a hard material to easily stick through the outer portion 73 made of a flexible material and to be securely caught in the inner portion 72 made of a hard material. In this way, the head portions of the pins on one of the rings facing with each other are securely caught in the inner portion of the other ring, and thereby the two rings are securely joined. Fig. 11 (a) is a plan view of a ring with pins having a structure described above.

Further, for the prevention of slippage or falling of the ring during deformation, the outer side surface of the ring may be processed to have one or more appropriate flat surfaces.

### Industrial Applicability

The present invention is useful as a device for connecting and conglutinating two blood vessels with different diameters.

### Reference Signs List

- 1: Small-diameter blood vessel
- 2: Incision
- 3: Large-diameter blood vessel
- 4: Blood vessel
- 4a: End portion of blood vessel
- 5: Truly circular ring
- 6: Vascular wall
- 7: Pin
- 8: Guide slit
- 9: Forceps
- 9a: Tweezers
- 10: Anastomosis device
- 10a: Guide plate
- 10b: Guide groove
- 11a, 11b: Ring with pins
- 12: Large-diameter blood vessel
- 13: Vascular wall
- 14: Pin
- 15: Small-diameter blood vessel
- 16: Incision
- 17: Vascular wall
- 18: Pin
- 21: Ring with pins
- 22: Ring holder
- 22a: Guide groove
- 23: Cylinder
- 24: Rotary knob
- 25: Support bar
- 25a: Protrusion
- 25b: Guide slit
- 26: Cap
- 27: Slot
- 28a, 28b: Cartridge component
- 29: Spring
- 30: Ring holder
- 30a: Guide groove
- 31: Ring with pins
- 32: Outer frame
- 33: Movable retainer plate
- 34: Spring
- 35: Fixed retainer plate
- 36: Tapered threaded portion
- 37: Bar
- 38: Tapered threaded portion
- 39: Driver
- 40: Protrusion
- 41a, 41b: Cartridge component
- 42: Spring
- 43: Ring holder
- 43a: Guide groove
- 44: Ring with pins
- 45: Outer frame
- 46: Thread
- 47: Movable retainer plate
- 48: Spring
- 49: Fixed retainer plate
- 50a,50b: Cartridge component
- 51: Spring
- 52: Bevel gear
- 53: Bar
- 54: Driver
- 55: Operation shaft
- 56: Bevel gear
- 57: Rod
- 58: Threaded portion
- 59: Movable retainer plate
- 60: Outer frame
- 61: Fixed retainer plate
- 62: Stopper
- 63: Ring holder
- 63a: Guide groove
- 64: Ring with pins
- 65: Guide member
- 66: Ball bearing
- 67,68,69,70: Pin
- 71: Ring
- 72: Inner portion
- 73: Outer portion
- 74: Flat surface

## Claims

1. A vascular anastomosis device for anastomosing two blood vessels with different diameters,
the device comprising
a first ring with pins and a second ring with pins both made of a material which is deformable by an external force and allows the pins to stick thereinto; and
an openable ring holder, which is made of a pair of components, detachably holds the first ring with pins and the second ring with pins, and has a means for deforming the rings with pins,
and being **characterized in that** vascular anastomosis can be achieved in the following manner:
the ring holder holding the rings is opened,
an end portion of a large-diameter blood vessel is inserted through the first ring and the vascular wall is extended outward 90 degrees and impaled on the pins,
an end portion of a small-diameter blood vessel is inserted through the second ring,
an incision is made in a longitudinal direction from the end surface of the small-diameter blood vessel,
the first ring and the second ring are deformed into a same elliptical shape by the means for deforming the rings so that the minor axis agrees with the diameter of the small-diameter blood vessel,
the small-diameter blood vessel is elliptically opened and the vascular wall is extended outward 90 degrees and impaled on the pins, and
the first ring and the second ring are joined together by allowing the pins provided on the ring in one component to stick into the ring in the other component to anastomose the large-diameter blood vessel and the small diameter blood vessel.

2. The vascular anastomosis device of claim 1, wherein the material which is deformable by an external force and allows the pins to stick thereinto is a flexible material.

3. The vascular anastomosis device of claim 1 or 2, wherein the pins have a harpoon-like shape having a barb.

4. The vascular anastomosis device of any one of claims 1 to 3, wherein the pins and the inner portion of the ring are made of a hard material, and the outer portion and other portions of the ring are made of a flexible material.

5. The vascular anastomosis device of any one of claims 1 to 4, wherein the rings are applied to human blood vessels, are truly circular, and have a thickness of 0.5 to 10 mm, an inner diameter of 0.3 to 50 mm, and a difference between the outer diameter and the inner diameter of 0.6 to 20 mm.

6. The vascular anastomosis device of any one of claims 1 to 4, wherein the rings are applied to human blood vessels, are elliptical, and have a minor axis of 0.3 to 50 mm and a difference between the outer diameter and the inner diameter of 0.6 to 20 mm.

7. The vascular anastomosis device of any one of claims 1 to 6, wherein the outer side surface of the rings are processed to partially have a flat surface as a means for the prevention of slippage or falling of the rings during ring deformation.

8. The vascular anastomosis device of any one of claims 1 to 7, wherein the means for deforming the rings is provided with a scale mark corresponding to the deformation amount of the ring deformation means.

9. A vascular anastomosis method for anastomosing two blood vessels with different diameters, **characterized in that**
a first ring with pins and a second ring with pins both made of a material which is deformable by an external force and allows the pins to stick thereinto are provided; and that
an end portion of the large-diameter blood vessel is inserted through the first ring and the vascular wall is extended outward 90 degrees and impaled on the pins,
an end portion of the small-diameter blood vessel is inserted through the second ring,
an incision is made in a longitudinal direction from the end surface of the small-diameter blood vessel,
the first ring and the second ring are deformed into a same elliptical shape by a means for deforming the rings so that the minor axis agrees with the diameter of the small-diameter blood vessel,
the small-diameter blood vessel is elliptically opened and the vascular wall is extended outward 90 degrees and impaled on the pins, and
the first ring and the second ring are joined together by allowing the pins provided on the ring in one component to stick into the ring in the other component to anastomose the large-diameter blood vessel and the small diameter blood vessel.
